# EUROPÄISCHE PATENTSCHRIFT

(11) **EP 2 955 176 B1**
(45) Veröffentlichungstag und Bekanntmachung des Hinweises auf die Patenterteilung: **06.12.2017**
(21) Anmeldenummer: 15171321.1
(22) Anmeldetag: 10.06.2011
(51) Int. Cl.: C07D 401/14, A01N 43/713

(54) **ZUSAMMENSETZUNG ENTHALTEND KRISTALLINE MODIFIKATION B VON (3-CHLOROPYRIDIN-2-YL)-N-[4-CYANO-2-METHYL-6-(METHYLCARBAMOYL)PHENYL]-3-([5-(TRIFLUORMETHYL)-2H- TETRAZOL-2-YL]METHYL)-1H-PYRAZOL-5-CARBOXAMID UND IHRE VERWENDUNG**
COMPOSITION COMPRISING CRYSTALLINE FORM B OF 1- (3-CHLOROPYRIDIN-2-YL)-N-[4-CYANO-2-METHYL-6-(METHYLCARBAMOYL)PHENYL]-3-([5-(TRIFLUOROMETHYL)-2H- TETRAZOL-2-YL]METHYL)-1H-PYRAZOL-5-CARBOXAMIDE AND ITS USE
COMPOSITION COMPRENANT FORME CRISTALLINE B DE 1- (3-CHLOROPYRIDIN-2-YL)-N-[4-CYANO-2-METHYL-6-(METHYLCARBAMOYL)PHENYL]-3-([5-(TRIFLUOROMETHYL)-2H- TETRAZOL-2-YL]METHYL)-1H-PYRAZOL-5-CARBOXAMIDE ET SON UTILISATION

(30) Priorität: 15.06.2010 EP 10166059; 15.06.2010 US 354920 P
(43) Veröffentlichungstag der Anmeldung: 16.12.2015
(62) Teilanmeldung aus: 11726391.3
(73) Patentinhaber: Bayer Intellectual Property GmbH, 40789 Monheim am Rhein (DE)
(72) Erfinder: PAZENOK, Dr. Sergii, 42699 Solingen (DE); LUI, Dr. Norbert, 51519 Odenthal (DE); VOLZ, Dr. Franz, 50825 Köln (DE); OLENIK, Dr. Britta, 46242 Bottrop (DE); FUNKE, Dr. Christian, 42799 Leichlingen (DE); FISCHER, Dr. Rüdiger, 50259 Pulheim (DE); GAERTZEN, Dr. Oliver, 50931 Köln (DE); HINZ, Martin-Holger, 42499 Hückeswagen (DE); NEEFF Arnd, 51399 Burscheid (DE)
(74) Vertreter: BIP Patents

(56) Entgegenhaltungen:
- WO-A2-2010/069502

## Beschreibung

Die vorliegende Erfindung betrifft eine agrochemische Zusammensetzung umfassend eine kristalline Modifikation B der Verbindung der Formel (I-1) sowie zusätzlich mindestens einen Hilfsstoff.

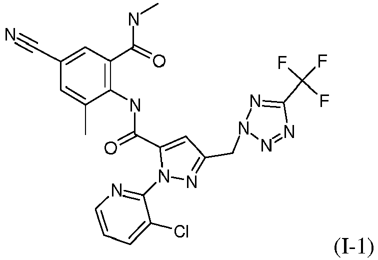

In der Literatur wurde bereits beschrieben, dass beispielsweise die Alkylierung von Alkyltetrazolen mit zum Beispiel Alkyliodiden üblicherweise zu einer Mischung aus verschiedenen Regioisomeren führt. Darüberhinaus hängt die Zusammensetzung des Gemisches stark von den jeweiligen Substituenten am Tetrazolring ab. So beschreibt William P. Norris, in J. Org. Chem., 1962, 27 (9), 3248-3251, dass bei der Alkylierung von 5-Trifluormethyltetrazol mit Methyljodid eine Mischung von beiden Regioisomeren in Verhältnis 6:1 entsteht. Die Alkylierung von Alkyltetrazolen führt dagegen meistens zu 1-substituerten Tetrazolen (siehe R-A.Henry et al, JACS, 76, 923 (1954).

Ebenfalls bekannt ist, dass das Auftreten von Wirkstoffen in verschiedenen kristallinen Modifikationen (Polymorphie) sowohl für die Ausarbeitung von Herstellungsverfahren als auch für die Entwicklung von Formulierungen von großer Bedeutung ist. So unterscheiden sich die verschiedenen kristallinen Modifikationen einer chemischen Verbindung neben dem Aussehen (Kristallhabitus) und der Härte auch in zahlreichen weiteren physiko-chemischen Eigenschaften. Dabei können Unterschiede bezüglich der Stabilität, der Filtrierbarkeit, der Löslichkeit, der Hygroskopizität, des Schmelzpunktes, der Feststoffdichte und der Fließfähigkeit einen starken Einfluss auf die Qualität und die Wirksamkeit von Pflanzenbehandlungsmitteln ausüben. Es ist bisher nicht möglich, das Auftreten und die Anzahl von kristallinen Modifikationen einschließlich ihrer physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Die Aufgabe der vorliegenden Erfindung ist weiterhin die Bereitstellung einer neuen stabilen agrochemischen Zusammensetzung umfassend eine kristalline Modifikation von einem Anthranilsäurediamid-Derivat.

Die Aufgabe wurde gemäß der vorliegenden Erfindung gelöst durch eine agrochemische Zusammensetzung umfassend die kristalline Modifikation B der Verbindung der Formel (I-1) 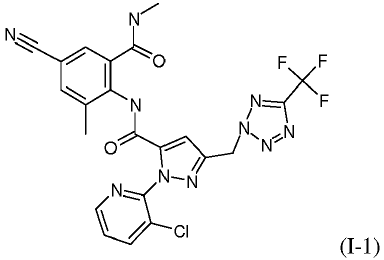 dadurch gekennzeichnet, dass ihr Röntgen-Pulverdiffraktogramm bei Verwendung von Cu Kα Strahlung mindestens folgende Reflexlagen aufweist:

| **2Theta/°** |
|---|
| 5.7 |
| 6.4 |
| 11.4 |
| 17.6 |
| 18.9 |
| 21.1 |
| 23.2 |
| 23.4 |
| 23.5 |
| 25.2 |

wobei die agrochemische Zusammensetzung zusätzlich mindestens einen Hilfsstoff ausgewählt aus Streckmitteln, Lösungsmitteln und/oder festen Trägerstoffen und/oder oberflächenaktiven Stoffen enthält.

Die erfindungsgemäß verwendeten Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E-als auch die Z-Isomeren, wie auch die threo- und erythro-, sowie die optischen Isomeren, beliebige Mischungen dieser Isomeren, sowie die möglichen tautomeren Formen offenbart

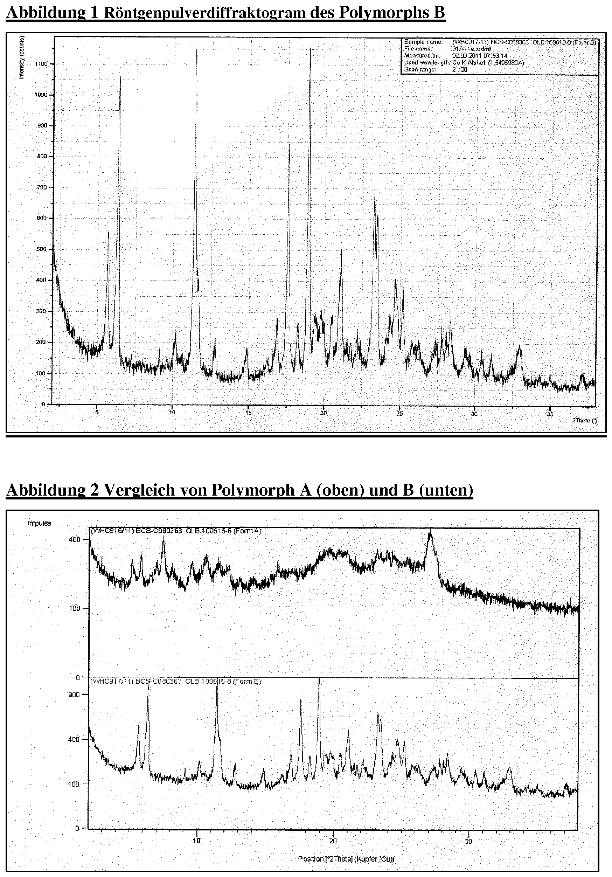

Die Verbindung der Formel (I-1) ist aus WO2010/069502 bekannt. Bislang war von der Verbindung 1-(3-Chloropyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid der Formel (I-1) nur eine Kristallform bekannt, die im Folgenden als Modifikation A bezeichnet wird. Modifikation A hat ein charakteristisches Röntgenpulverdiffraktogramm, Raman-Spektrum und IR-Spektrum (Tab. 1-2, Abb. 2, 3, 5). Diese kristalline Modifikation ist thermodynamisch metastabil und fällt in Form blockartiger Kristalle an.

Die kristalline Modifikation B ist dadurch gekennzeichnet, dass sie ein Röntgenpulverdiffraktogramm mit den in Tabelle 1 angegebenen Reflexlagen (2 Theta) aufweist. Das Röntgenpulverdiffraktogramm der kristallinen Modifikation B ist auch in der Abbildung 1 wiedergegeben. Die intensivsten Signale (2 Theta) des Röntgenpulver-diffraktogramms der kristallinen Modifikation B liegen demnach bei 5.7°, 6.4°, 11.4°, 17.6°, 18.9°, 21.1°, 23.2°, 23.4°, 23.5° und 25.2° (jeweils ± 0,2°)

Alle Röntgenpulverdiffraktometrie-Daten der Modifikation B wurden mit folgenden Akquisitionsparametern erhalten:

| | |
|---|---|
| Diffraktometer Typ: | PANalytic X' Pert PRO |
| Anodenmaterial: | Cu |
| Wellenlänge: | 1.54060 |
| Scan Modus: | Transmission |
| Scan Typ: | 2Theta:Omega |
| Angabe 2Theta: | ± 0,2° |

Die bekannte kristalline Modifikation A der Verbindung der Formel (1) ist dadurch gekennzeichnet, dass sie ein Röntgenpulverdiffraktogramm mit den in der folgenden Tabelle 1 angegebenen Reflexlagen (2 Theta) aufweist. Das Röntgenpulverdiffraktogramm der kristallinen Modifikation A ist auch in der Abbildung 2 wiedergegeben.

Alle Röntgenpulverdiffraktometrie-Daten der Modifikation A wurden ebenfalls mit folgenden Akquisitionsparametern erhalten:

| | |
|---|---|
| Diffraktometer Typ: | PANalytic X' Pert PRO |
| Anodenmaterial: | Cu |
| Wellenlänge: | 1.54060 |
| Scan Modus: | Transmission |
| Scan Typ: | 2Theta:Omega |
| Angabe 2Theta: | ± 0,2° |

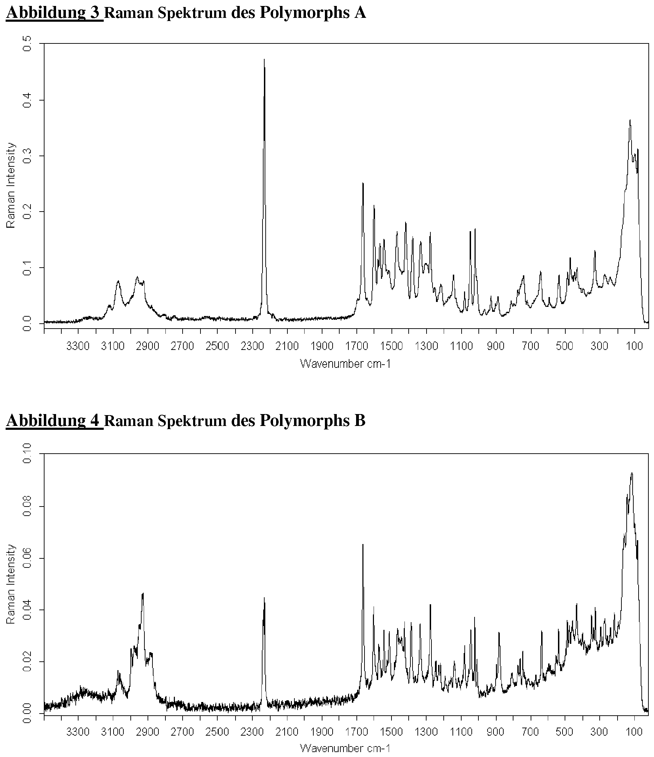

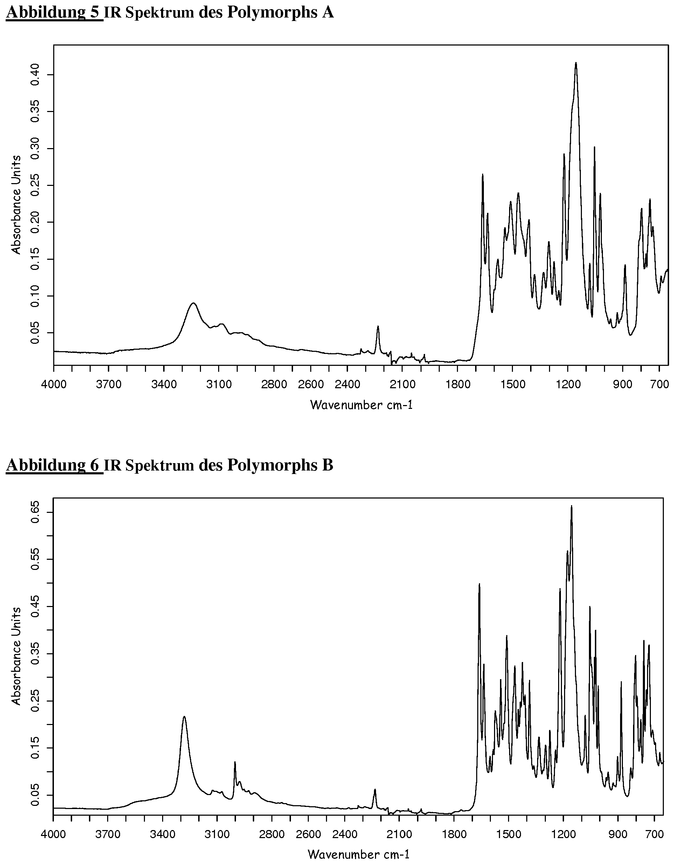

Die erfindungsgemäße kristalline Modifikation B der Verbindung der Formel (I-1) kann außerdem durch IR- und Raman-Spektroskopie charakterisiert werden. Die entsprechenden Raman- und IR-Spektren sind in Abbildung 4 und 6 abgebildet.

Die IR- und Raman-Spektren enthalten folgende Banden der kristallinen Modifikationen A und B, die in Tabelle 2 aufgelistet sind.

### Herstellbeispiele

Die folgenden Herstellbeispiele illustrieren die Erfindung, ohne sie zu beschränken.

### Beispiel 1

### Isomeren Gemisch von Methyl 5-(3-chloropyridin-2-yl)-3-{[5-(trifluoromethyl)-2H-tetrazol-2-yl]methyl}cyclopenta-1,3-diene-1-carboxylate (Hauptisomer) und Methyl 5-(3-chloropyridin-2-yl)-3-{[5-(trifluoromethyl)-1H-tetrazol-2-yl]methyl}cyclopenta-1,3-diene-1-carboxylate (Nebenkomponente)

2.86 g (0.01 mol) von Methyl 3-(chlormethyl)-1-(3-chloropyridin-2-yl)-1H-pyrazole-5-carboxylate und 1,6 g von Natrium 5-(trifluoromethyl)tetrazol-2-ide und 0.15 g KI in 50 ml Aceton wurden 9 Std bei 56 °C erhitzt. Die Salze wurden abfiltriert und Aceton in Vakuum entfernt. Man erhielt 4.59 g des Produktes als 9:1 Gemisch von beiden Isomeren.

### Analytische Charakterisierung

### Hauptisomer

¹H NMR (CD₃CN) δ: 8,52 (1H, d); 7.95 (1H,d), 7,45 (1H, dd); 7,10 (1H, s); 6.05 (2H,s) 3,75 (3H, s) ppm.
¹⁹F NMR -64.05 ppm.

### Nebenkomponente

¹⁹F NMR-61.46 ppm.
¹H NMR (CD₃CN) δ: 8,50 (1H, d); 7.90 (1H,d), 7,45 (1H, dd); 6,95 (1H, s); 5,80 (2H,s) 3,70 (3H, s) ppm.

### Beispiel 2

### 1-(3-chloropyridin-2-yl)-3-{[5-(trifluoromethyl)-2H-tetrazol-2-yl]methyl}-1H-pyrazole-5-carboxylic acid (Hauptisomer) und 1-(3-Chloropyridin-2-yl)-3-[5-(trifluoromethyl)-1H-tetrazol-1-yl]-1H-pyrazole-5-carboxylic acid (Nebenkomponente)

4.59 g des Gemisches aus dem Beispiel 1 wurden in 40 ml Methanol gelöst und 2 g NaOH als 10 % Lösung in Wasser wurden zugegeben. Das Gemisch wurde 3 Std. bei RT gerührt.

10 % iger HCl wurde zugegeben, um den pH der Lösung auf 3 einzustellen und das Produkt wurde mit Methyltert.butylether extrahiert. Nach der Entfernung des Lösemittels wird der Rückstand (4 g) ohne Aufreinigung weiter umgesetzt.

### Analytische Charakterisierung Hauptisomer 90 %

¹H NMR (CD₃CN) δ: 13,5 (b.s) 8,52 (1H, d); 8,2 (1H,d), 7,6 (1H, dd); 7,2 (1H, s); 6.25 (2H,s) ppm.
¹⁹F NMR 64.25 - ppm.

### Beispiel 3

### Isomeren Gemisch von 1-(3-chloropyridin-2-yl)-N-[4-cyano-2-methyl-6-(methylcarbamoyl)-phenyl]-3-{[5-(trifluoromethyl)-2H-tetrazol-2-yl]methyl}-1H-pyrazole-5-carboxamide (Hauptisomer) und 1-(3-chloropyridin-2-yl)-N-[4-cyano-2-methyl-6-(methylcarbamoyl)phenyl]-3-[5-(trifluoro-methyl)-1H-tetrazol-1-yl]-1H-pyrazole-5-carboxamide (Nebenkomponente) im Verhältnis 94:6.

10 g von 1-(3-chloropyridin-2-yl)-3-{[5-(trifluoromethyl)-2H-tetrazol-2-yl]methyl}-1H-pyrazole-5-carboxylic acid und 1-(3-Chloropyridin-2-yl)-3-[5-(trifluoromethyl)-1H-tetrazol-1-yl]-1H-pyrazole-5-carboxylic acid als 90:10 Gemisch wurden in 40 ml Dimethylacetamid vorgelegt. 4.75 g von 2-Amino-5-cyano-N,3-dimethylbenzamide, 7.2 g 2,6-Dimethylpyridine und 3.9 g von Mesylchlorid wurden zugegeben und das Gemisch wurde 4 Std bei 50°C erhitzt. Das Gemisch wurde auf 20°C abgekühlt und 100 ml Wasser wurden zugegeben. Nach ca, 1 Std der Niederschlag wurde abfiltriert, mit Wasser gewaschen und gtetrocknet.. Man erhält 12.1 g des Produktes (93 % Ausbeute) mit isomeren Verhältniss 94:6. und einer Reinheit von 95-96 %.

### Analytische Charakterisierung

**Hauptisomer 94 %**

| H/C | δH/ppm | Mult. | rel. No. H | δC/ppm | Mult. | rel. No. C |
|---|---|---|---|---|---|---|
| 1 | - | - | - | 118.7 | Q | 1 |
| 2 | - | - | - | 156.1 | Q | 1 |
| 3 | 6.34 | S | 2 | 51.3 | T | 1 |
| 4 | - | - | - | 145.6 | S | 1 |
| 5 | 7.40 | S | 1 | 108.5 | D | 1 |
| 6 | - | - | - | 138.8 | S | 1 |
| 7 | - | - | - | 156.3 | S | 1 |
| 8 | 10.55 | S | 1 | - | - | - |
| 9 | - | - | - | 137.6 | S | 1 |
| 10 | - | - | - | 138.7 | S | 1 |
| 11 | - | - | - | 166.2 | S | 1 |
| 12 | 8.38 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.4 | S | 1 |
| 16 | - | - | - | 118.3 | S | 1 |
| 17 | 7.87 | D | 1 | 135.2 | D | 1 |
| 18 | - | - | - | 138.0 | S | 1 |
| 19 | 2.20 | S | 3 | 18.0 | Q | 1 |
| 20 | - | - | - | 149.1 | S | 1 |
| 21 | - | - | - | 128.0 | S | 1 |
| 22 | 8.16 | DD | 1 | 139.4 | D | 1 |
| 23 | 7.60 | DD | 1 | 126.7 | D | 1 |
| 24 | 8.48 | DD | 1 | 147.3 | D | 1 |

**Nebenkomponente**

| H/C | δH/ppm | Mult. | rel. No. H | δC/ppm | Mult. | rel. No. C |
|---|---|---|---|---|---|---|
| 1 | - | - | - | 118.1 | Q | 1 |
| 2 | - | - | - | 145.9 | Q | 1 |
| 3 | 6.11 | S | 2 | 47.0 | T | 1 |
| 4 | - | - | - | 145.9 | S | 1 |
| 5 | 7.35 | S | 1 | 107.7 | D | 1 |
| 6 | - | - | - | 138.8 | S | 1 |
| 7 | - | - | - | 156.2 | S | 1 |
| 8 | 10.54 | S | 1 | - | - | - |
| 9 | - | - | - | 137.6 | S | 1 |
| 10 | - | - | - | 135.2 | S | 1 |
| 11 | - | - | - | 166.2 | S | 1 |
| 12 | 8.37 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.3 | S | 1 |
| 16 | - | - | - | 118.3 | S | 1 |
| 17 | 7.87 | D | 1 | 135.4 | D | 1 |
| 18 | - | - | - | 138.0 | S | 1 |
| 19 | 2.19 | S | 3 | 17.9 | Q | 1 |
| 20 | - | - | - | 149.1 | S | 1 |
| 21 | - | - | - | 128.1 | S | 1 |
| 22 | 8.14 | DD | 1 | 139.4 | D | 1 |
| 23 | 7.58 | DD | 1 | 126.7 | D | 1 |
| 24 | 8.47 | DD | 1 | 147.2 | D | 1 |

### Beispiel 4

### 1-(3-chloropyridin-2-yl)-3-{[5-(trifluoromethyl)-2H-tetrazol-2-yl]methyl}-1H-pyrazole-5-carbonsäure (Hauptisomer) und 1-(3-Chloropyridin-2-yl)-3-[5-(trifluoromethyl)-1H-tetrazol-1-yl]-1H-pyrazole-5-carbonsäure (Nebenkomponente)

105 g (0.36 mol) von Methyl 3-(chlormethyl)-1-(3-chloropyridin-2-yl)-1H-pyrazole-5-carboxylate mit der Reinheit von 98 %, 204 g (0.384 mol) von Natrium 5-(trifluormethyl)tetrazol-2-ide (30 % Lösung in Aceton) und 24 g (0.144 mol) KI in 680 ml Aceton wurden 10 Std bei 56 °C erhitzt. Die Salze wurden abfiltriert und Aceton in Vakuum entfernt. Das Produkt (Öl) wurde in 300 ml Toluol aufgenommen und die Lösung mit 100 ml Wasser gewaschen. Die toluolische Lösung wurde dann mit 170 g 10 %iger Lösung NaOH 6 Std bei bei 40°C gerührt. Die organische Phase wurde abgetrennt und die wässrige Phase wurde mit 10 % HCl auf pH 3 langsam gestellt. Das ausgefallen Produkt wurde abfiltriert, mit Wasser gewaschen und getrocknet. Man erhielt 118 g (85 % Ausbeute) des Produktes mit einer w.w. % Reinheit von 97.3 %. Verhältnis von Regioisomeren 94:6.

Die vorliegende Erfindung betrifft Formulierungen und daraus bereitete Anwendungsformen als Pflanzenschutzmittel und/oder Schädlingsbekämpfungsmittel wie z. B. Drench-, Drip- und Spritzbrühen. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanolalkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen die vorliegende Erfindung betreffend sind beispielsweise Suspensionskonzentrate (SC, SE, FS, OD), in Wasser dispergierbare Granulate (WG), und Granulate (GR); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

Die folgenden Beispiele illustrieren die Erfindung ohne sie einzuschränken. Die in folgenden Beispielen eingesetzten Lösungsmittelsysteme sind besonders bevorzugt. Die erfindungsgemäße Verbindung der Formel (I-1) wurde in drei verschiedenen Formulier-Rezepturen mit drei unterschiedlichen Konzentrationen zu wasserbasierten Suspensionskonzentraten verarbeitet (Formulierbeispiele 1, 2 und 3). Dabei wurde die Verbindung der Formel (I-1) als Wirkstoff sowohl in der Form A als auch in der Form B eingesetzt. Anschließend wurden die hergestellten Suspensionskonzentrate unter gleichen Bedingungen gelagert und nach Ende der Lagerung auf ihre Eigenschaften hin untersucht. Überraschenderweise wurde gefunden, dass die mit der Verbindung der Formel (I-1) als Form B hergestellten Suspensionskonzentrate sich in ihren Eigenschaften erheblich von den Suspensionskonzentraten unterschieden, die mit der Verbindung der Formel (I-1) als Form A hergestellt worden waren.

### Pormulierbeispiel

### Beispiel 1

Zur Herstellung eines Suspensionskonzentrates werden bei Raumtemperatur

| | | |
|---|---|---|
| 7,28 | Gew.-% | Verbindung (I-1) in Form A |
| 0,20 | Gew.-% | Wässrige Siliconöl-Emulsion, z.B. Silfoam SRE (Fa. Wacker) |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 0,50 | Gew.-% | Natrium-Alkylnaphthalinsulfonat-Formaldehydkondensat, z.B. Morwet D-425 (Fa. Akzo-Nobel) |
| 10,00 | Gew.-% | Glycerin |
| 4,00 | Gew.-% | Ethoxyliertes Polymethacrylat, z.B. Atlox 4913 (Fa. Croda) |
| 1,50 | Gew.-% | Tristyrylphenolethoxylat, z.B. Tanemul PS 54 (Fa. Tanatex) |
| 56,02 | Gew.-% | entmineralisiertes Wasser |

zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,10 | Gew.-% | Xanthan-Verdicker, z.B. Kelzan S (Fa. CP Kelco) |
| 20,00 | Gew.-% | entmineralisiertes Wasser |

aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (I-1) in der Form B enthält.

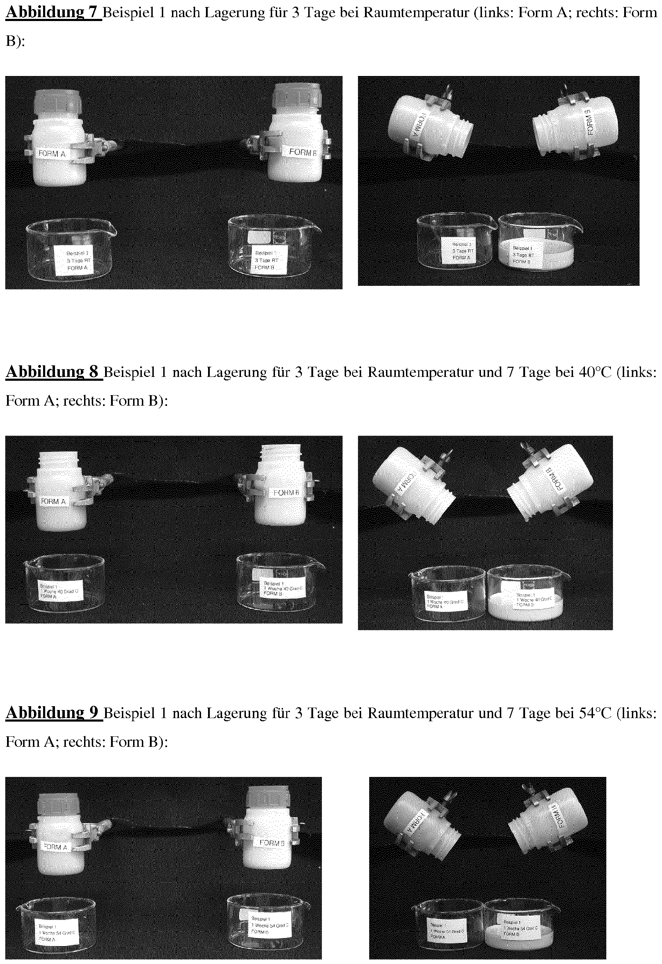

Beide Suspensionskonzentrate (Form A und Form B) sind direkt nach Herstellung homogen und dünnflüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht, ggf. durch leichtes Schütteln homogenisiert und verglichen (siehe Abbildung 7-10).

### Beispiel 2

Zur Herstellung eines Suspensionskonzentrates werden bei Raumtemperatur

| | | |
|---|---|---|
| 8,47 | Gew.-% | Verbindung (I-1) in Form A |
| 0,20 | Gew.-% | Wässrige Siliconöl-Emulsion, z.B. Silfoam SRE |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 0,50 | Gew.-% | Natrium-Alkylnaphthalinsulfonat-Formaldehydkondensat, z.B. Morwet D-425 |
| 10,00 | Gew.-% | 1,2-Propandiol |
| 1,00 | Gew.-% | EO-PO-Block-Copolymerisat, z.B. Synperonic PE/F 127 (Fa. Croda) |
| 4,00 | Gew.-% | Tristyrylphenolethoxylat-Sulfat, z.B. Soprophor 4D384 (Fa. Rhodia) |
| 0,50 | Gew.-% | Amorphe gefällte Kieselsäure, z.B. Sipernat 22S (Fa. Evonik) |
| 54,83 | Gew.-% | entmineralisiertes Wasser |

Zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,10 | Gew.-% | Xanthan-Verdicker, z.B. Kelzan S |
| 20,00 | Gew.-% | entmineralisiertes Wasser |

Aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (1) in der Form B enthält.

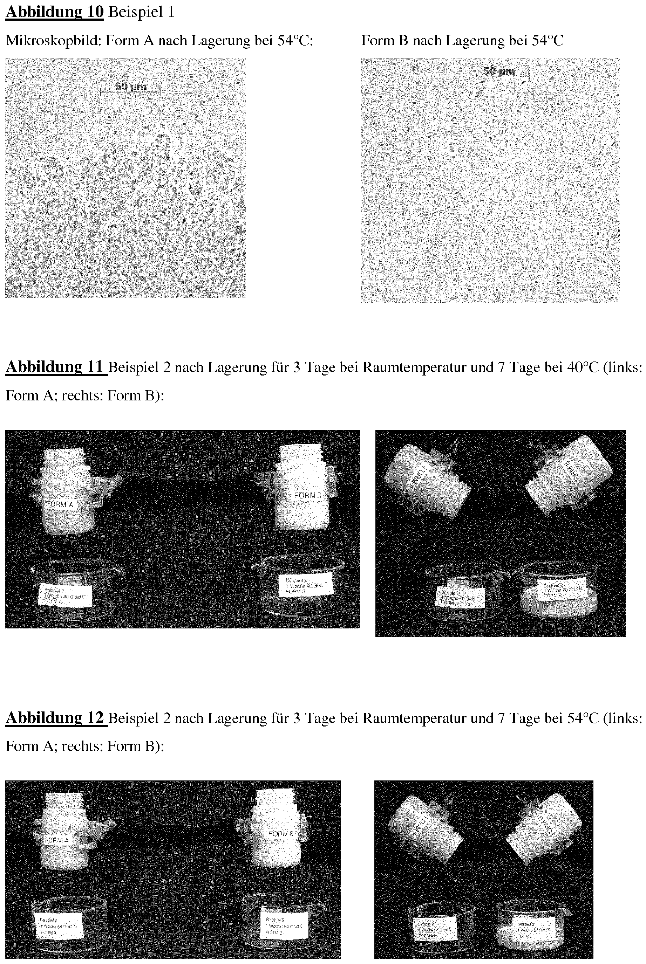

Beide Suspensionskonzentrate (Form A und Form B) sind direkt nach Herstellung homogen und dünnflüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht, ggf. durch leichtes Schütteln homogenisiert und verglichen (siehe Abbildung 11-13).

Beispiel 2 nach Lagerung für 3 Tage bei Raumtemperatur:
Nach 3 Tagen Lagerung bei Raumtemperatur sind beide Proben homogen und dünnflüssig.

### Beispiel 3

Zur Herstellung eines erfindungsgemäßen Suspensionskonzentrates werden bei Raumtemperatur

| | | |
|---|---|---|
| 4,85 | Gew.-% | Verbindung (I-1) in Form A |
| 0,20 | Gew.-% | Wässrige Siliconöl-Emulsion, z.B. Silfoam SRE |
| 0,20 | Gew.-% | Zitronensäure |
| 0,12 | Gew.-% | Wässrige Benzoisothiazolinon-Lösung, z.B. Proxel GXL 20% |
| 0,08 | Gew.-% | Gemisch aus Bioziden vom Isothiazolon-Typ in wässriger Lösung, z.B. Preventol D7 |
| 1,50 | Gew.-% | Natrium-Alkylnaphthalinsulfonat-Formaldehydkondensat, z.B. Morwet D-425 |
| 10,00 | Gew.-% | 1,2-Propandiol |
| 6,25 | Gew.-% | Ethoxyliertes Polymethacrylat, z.B. Atlox 4913 |
| 2,00 | Gew.-% | Butyl-EO-PO-Block-Copolymer, z.B. Atlas G-5000 (Fa. Croda) |
| 54,60 | Gew.-% | entmineralisiertes Wasser |

Zusammengegeben, und nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen. Danach wird mit

| | | |
|---|---|---|
| 0,20 | Gew.-% | Xanthan-Verdicker, z.B. Kelzan S |
| 20,00 | Gew.-% | entmineralisiertes Wasser |

Aufgefüllt; dabei wird das Kelzan S als 2%ige Vormischung eingesetzt. Anschließend wird die Suspension solange nachgerührt bis eine homogene Suspension entstanden ist.

Analog der oben genannten Versuchsvorschrift wird ein Suspensionskonzentrat hergestellt die die Verbindung (I-1) in der Form B enthält.

Beide Suspensionskonzentrate (Form A und Form B) sind direkt nach Herstellung homogen und flüssig. Teilmengen beider Suspensionskonzentrate werden abgefüllt und zunächst 3 Tage bei Raumtemperatur stehen gelassen und danach für 7 bzw. 14 Tage bei 40°C und 54°C gelagert. Anschließend werden die Probenflaschen auf Raumtemperatur gebracht und verglichen.

Beispiel 3 nach Lagerung für 3 Tage bei Raumtemperatur:
Nach 3 Tagen Lagerung bei Raumtemperatur sind beide Proben homogen und dünnflüssig

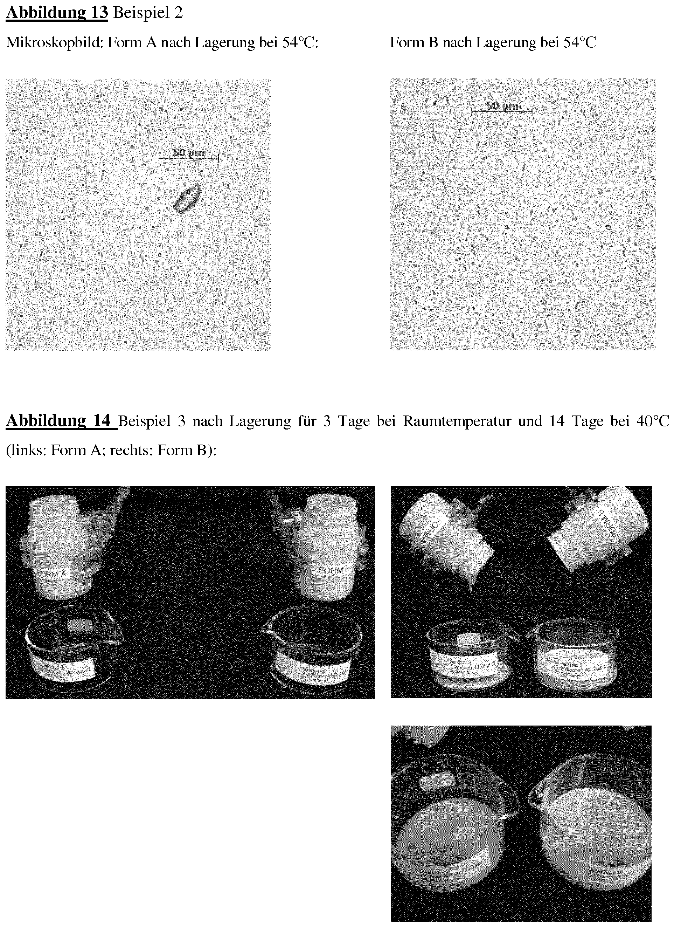

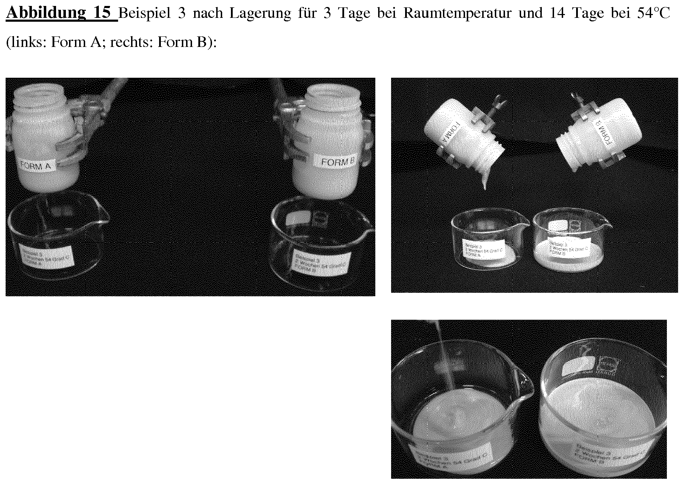

Beispiel 3 nach Lagerung für 3 Tage bei Raumtemperatur und 7 Tage bei 40°C bzw. 54°C:
Nach 3 Tagen Lagerung bei Raumtemperatur und 7 Tagen bei 40°C bzw. 54°C sind beide Proben dünnflüssig, zeigen aber eine leichte Phasentrennung (siehe Abbildung 14-15).

**Tabelle 1: Röntgenpulverdiffraktometrie**

| **Reflexe [2 Theta]** | |
|---|---|
| **Modifikation A** | **Modifikation B** |
| 2.9 | 5.7 |
| 5.2 | 6.4 |
| 5.9 | 7.3 |
| 6.5 | 8.3 |
| 7.0 | 9.1 |
| 7.3 | 9.6 |
| 7.4 | 10.2 |
| 8.1 | 10.6 |
| 9.6 | 11.4 |
| 10.6 | 11.7 |
| 11.6 | 12.8 |
| 12.3 | 14.9 |
| 13.1 | 16.2 |
| 14.2 | 16.8 |
| 15.9 | 17.6 |
| 18.8 | 18.2 |
| 19.6 | 18.9 |
| 20.3 | 19.3 |
| 21.0 | 19.7 |
| 22.1 | 20.0 |
| 22.7 | 20.5 |
| 23.1 | 21.1 |
| 23.8 | 21.5 |
| 24.3 | 21.8 |
| 25.4 | 22.2 |
| 25.8 | 22.9 |
| 26.9 | 23.2 |
| 27.5 | 23.4 |
| 31.3 | 23.5 |
| 32.4 | 24.0 |
| 37.2 | 24.3 |
| | 24.6 |
| | 25.2 |
| | 25.8 |
| | 25.8 |
| | 27.3 |
| | 27.8 |
| | 28.0 |
| | 28.3 |
| | 29.4 |
| | 29.7 |
| | 30.4 |
| | 31.0 |
| | 31.7 |
| | 32.2 |
| | 32.9 |
| | 34.3 |
| | 35.0 |
| | 37.1 |

**Tabelle 2: IR- und Raman-Banden**

| ***Modifikation A*** | ***Modifikation B*** | ***Modifikation A*** | ***Modifikation B*** |
|---|---|---|---|
| **IR-Banden [cm⁻¹]** | **IR-Banden [cm⁻¹]** | **Raman-Banden [cm⁻¹]** | **Raman-Banden [cm⁻¹]** |
| 3238 | 3280 | 3117 | 3073 |
| 3087 | 3002 | 3070 | 2997 |
| 2232 | 2977 | 2962 | 2929 |
| 1662 | 2233 | 2927 | 2881 |
| 1636 | 1661 | 2235 | 2231 |
| 1579 | 1637 | 2231 | 2236 |
| 1541 | 1602 | 1664 | 1664 |
| 1510 | 1574 | 1601 | 1602 |
| 1468 | 1543 | 1578 | 1574 |
| 1410 | 1510 | 1567 | 1543 |
| 1379 | 1466 | 1543 | 1512 |
| 1332 | 1446 | 1516 | 1464 |
| 1304 | 1421 | 1468 | 1424 |
| 1275 | 1410 | 1419 | 1386 |
| 1249 | 1385 | 1378 | 1335 |
| 1220 | 1334 | 1332 | 1275 |
| 1155 | 1298 | 1306 | 1244 |
| 1080 | 1275 | 1277 | 1229 |
| 1054 | 1244 | 1252 | 1219 |
| 1022 | 1219 | 1218 | 1141 |
| 930 | 1178 | 1147 | 1081 |
| 887 | 1155 | 1081 | 1044 |
| 797 | 1080 | 1048 | 1022 |
| 773 | 1055 | 1021 | 1008 |
| 751 | 1046 | 966 | 897 |
| 736 | 1029 | 929 | 882 |
| 690 | 1023 | 889 | 772 |
| | 1009 | 813 | 760 |
| | 954 | 775 | 745 |
| | 902 | 744 | 636 |
| | 882 | 645 | 539 |
| | 829 | 594 | 489 |
| | 803 | 537 | 475 |
| | 795 | 489 | 457 |
| | 774 | 474 | 350 |
| | 758 | 449 | 337 |
| | 743 | 434 | 327 |
| | 729 | 400 | 296 |
| | 669 | 331 | 271 |
| | | 274 | 218 |
| | | 245 | 115 |
| | | 129 | |
| | | 100 | |
| | | 84 | |

## Patentansprüche

1. Agrochemische Zusammensetzung umfassend die kristalline Modifikation B der Verbindung der Formel (I-1) 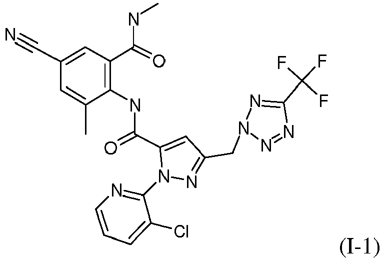 **dadurch gekennzeichnet, dass** ihr Röntgen-Pulverdiffraktogramm bei Verwendung von Cu Kα Strahlung mindestens folgende Reflexlagen aufweist:
| **2Theta/°** |
|---|
| 5.7 |
| 6.4 |
| 11.4 |
| 17.6 |
| 18.9 |
| 21.1 |
| 23.2 |
| 23.4 |
| 23.5 |
| 25.2 |
wobei die agrochemische Zusammensetzung zusätzlich mindestens einen Hilfsstoff ausgewählt aus Streckmitteln, Lösungsmitteln und/oder festen Trägerstoffen und/oder oberflächenaktiven Stoffen enthält.

2. Agrochemische Zusammensetzung gemäß Anspruch 1 , **dadurch gekennzeichnet, dass** das Röntgen-Pulverdiffraktogramm der Kristallinen Modifikation B der Verbindung (I-1) bei Verwendung von Cu Kα-Strahlung mindestens folgende weitere Reflexlagen aufweist:
| **2Theta/°** |
|---|
| 11.7 |
| 16.8 |
| 19.7 |
| 20.5 |
| 24.3 |
| 24.6 |
| 28.3 |

3. Agrochemische Zusammensetzung gemäß einem der Ansprüche 1-2, **dadurch gekennzeichnet, dass** das Raman-Spektrum der kristallinen Modifikation B der Verbindung der Formel (I-1) mindestens folgende Banden aufweist:
| **Bande [cm⁻¹]** |
|---|
| 2929 |
| 1386 |
| 882 |
| 636 |

4. Agrochemische Zusammensetzung gemäß einem der Ansprüche 1-3 umfassend ein oder mehrere Hilfsstoffe ausgewählt aus Streckmittel, Lösemittel, Spontanitätsförderer, Trägerstoffe, Emulgiermittel, Dispergiermittel, Frostschutzmittel, Biozide, Verdicker und/oder Adjuvantien.

5. Agrochemische Zusammensetzung gemäß einem der Ansprüche 1-4, **dadurch gekennzeichnet, dass** es sich um ein Suspensionskonzentrat handelt.

6. Verfahren zur Bekämpfung von unerwünschten Insekten, **dadurch gekennzeichnet, dass** eine agrochemische Zusammensetzung gemäß einem der Ansprüche 1-5 auf die Insekten und/oder ihren Lebensraum und/oder Saatgut ausgebracht wird, ausgenommen zur therapeutischen Behandlung des tierischen oder menschlichen Körper.

## Claims

1. Agrochemical composition comprising the crystal polymorph B of the compound of the formula (I-1) 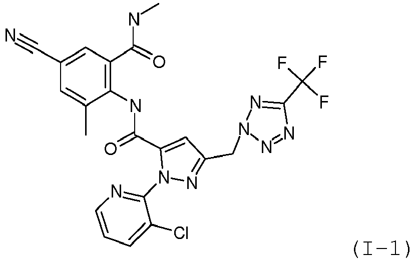 **characterized in that** the x-ray powder diffractogram thereof using Cu Kα radiation has at least the following reflections:
| **2theta / °** |
|---|
| 5.7 |
| 6.4 |
| 11.4 |
| 17.6 |
| 18.9 |
| 21.1 |
| 23.2 |
| 23.4 |
| 23.5 |
| 25.2 |
wherein the agrochemical composition additionally contains at least one auxiliary selected from extenders, solvents and/or solid carriers and/or surfactants.

2. Agrochemical composition according to Claim 1, **characterized in that** the x-ray powder diffractogram of the crystal polymorph B of the compound (I-1) using Cu Kα radiation has at least the following further reflections:
| **2theta / °** |
|---|
| 11.7 |
| 16.8 |
| 19.7 |
| 20.5 |
| 24.3 |
| 24.6 |
| 28.3 |

3. Agrochemical composition according to one of Claims 1-2, **characterized in that** the Raman spectrum of the crystal polymorph B of the compound of the formula (I-1) has at least the following bands:
| **Band [cm⁻¹]** |
|---|
| 2929 |
| 1386 |
| 882 |
| 636 |

4. Agrochemical composition according to one of Claims 1-3 comprising one or more auxiliaries selected from extenders, solvents, spontaneity promoters, carriers, emulsifiers, dispersants, frost protectants, biocides, thickeners and/or adjuvants.

5. Agrochemical composition according to one of Claims 1-4, **characterized in that** it is a suspension concentrate.

6. Method for controlling unwanted insects, **characterized in that** an agrochemical composition according to one of Claims 1-5 is applied to the insects and/or their habitat and/or seed, excluding for therapeutic treatment of the animal or human body.

## Revendications

1. Composition agrochimique comprenant la forme cristalline B du composé de formule (I-1) 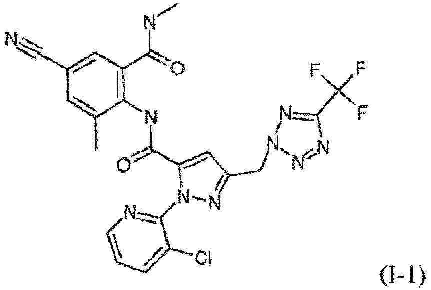 **caractérisée en ce que** son diffractogramme de rayons X sur poudre lors de l'utilisation d'un rayonnement Cu Kα comprend au moins les positions de réflexion suivantes :
| 2-Thêta/° |
|---|
| 5,7 |
| 6,4 |
| 11,4 |
| 17,6 |
| 18,9 |
| 21,1 |
| 23,2 |
| 23,4 |
| 23,5 |
| 25,2 |
la composition agrochimique contenant en outre au moins un auxiliaire choisi parmi les extendeurs, les solvants et/ou les véhicules solides et/ou les substances tensioactives.

2. Composition agrochimique selon la revendication 1, **caractérisée en ce que** le diffractogramme de rayons X sur poudre de la forme cristalline B du composé (I-1) lors de l'utilisation d'un rayonnement Cu Kα comprend au moins les positions de réflexion supplémentaires suivantes :
| 2-Thêta/° |
|---|
| 11,7 |
| 16,8 |
| 19,7 |
| 20,5 |
| 24,3 |
| 24,6 |
| 28,3 |

3. Composition agrochimique selon l'une quelconque des revendications 1 à 2, **caractérisée en ce que** le spectre de Raman de la forme cristalline B du composé de formule (I-1) comprend au moins les bandes suivantes :
| Bande [cm⁻¹] |
|---|
| 2929 |
| 1386 |
| 882 |
| 636 |

4. Composition agrochimique selon l'une quelconque des revendications 1 à 3, comprenant un ou plusieurs auxiliaires choisis parmi les extendeurs, les solvants, les promoteurs de spontanéité, les véhicules, les émulsifiants, les dispersants, les agents antigel, les biocides, les épaississants et/ou les adjuvants.

5. Composition agrochimique selon l'une quelconque des revendications 1 à 4, **caractérisée en ce qu'**il s'agit d'un concentré de suspension.

6. Procédé de lutte contre des insectes indésirables, **caractérisé en ce qu'**une composition agrochimique selon l'une quelconque des revendications 1 à 5 est appliquée sur les insectes et/ou leur habitat et/ou des graines, à l'exception du traitement thérapeutique du corps animal ou humain.
